# EUROPEAN PATENT APPLICATION

(11) **EP 3 488 789 A1**
(43) Date of publication of application: **29.05.2019**
(21) Application number: 18208634.8
(22) Date of filing: 27.11.2018
(51) Int. Cl.: A61B 17/00

(54) **SPECIMEN RETRIEVAL DEVICE**

(30) Priority: 28.11.2017 US 201762591704 P; 24.09.2018 US 201816139355
(71) Applicant: Covidien LP, Mansfield, MA 02048 (US)
(72) Inventor: GEORGE, Sabastian Koduthully, 50050 Hyderabad (IN); KAMARAJ, Raja, 500055 Hyderabad (IN)
(74) Representative: Maschio, Antonio

(57) **Abstract**

A specimen retrieval device includes a tubular body defining a longitudinal bore, an inner shaft slidably disposed within the longitudinal bore of the tubular body, and a specimen bag affixed to a support mechanism at the distal portion of the inner shaft. In embodiments, the specimen bag has an opening capable of being closed by an interlocking assembly after placement of tissue therein, but prior to removal of the device from the body.

## Description

### CROSS-REFERENCE TO RELATED APPLICATION

This application claims the benefit of and priority to U.S. Provisional Patent Application No. 62/591,704 filed November 28, 2017, the entire disclosure of which is incorporated by reference herein.

### TECHNICAL FIELD

The present disclosure relates generally to surgical apparatuses for use in minimally invasive surgical procedures, such as endoscopic and/or laparoscopic procedures and, more particularly, the present disclosure relates to specimen retrieval devices including an opening that may be closed after placement of a tissue specimen therein.

### BACKGROUND

Minimally invasive surgery, such as endoscopic surgery, reduces the invasiveness of surgical procedures. Endoscopic surgery involves surgery through body walls, for example, viewing and/or operating on the ovaries, uterus, gall bladder, bowels, kidneys, appendix, etc. There are many common endoscopic surgical procedures, including arthroscopy, laparoscopy, gastroentroscopy and laryngobronchoscopy, just to name a few. In these procedures, trocars are utilized for creating incisions through which the endoscopic surgery is performed. Trocar tubes or cannula devices are extended into and left in place in the abdominal wall to provide access for endoscopic surgical tools. A camera or endoscope is inserted through a trocar tube to permit visual inspection and magnification of a body cavity. The surgeon can then perform diagnostic and/or therapeutic procedures at the surgical site with the aid of specialized instrumentation, such as forceps, graspers, cutters, applicators, and the like, which are designed to fit through additional cannulas.

When removing certain tissues from the body cavity, for example tumor tissue, it is important that the tumor tissue does not come into contact with healthy or uninvolved tissue. If tumor tissue or tissue parts have to be removed, they may be introduced into an "extraction bag," also referred to herein as a "specimen bag," at the site where the tumor or diseased tissue has been detached from the surrounding tissue, after which the specimen bag is withdrawn from the body, thereby minimizing contact of the diseased tissue with healthy tissue.

Improved specimen bags for use in minimally invasive surgical procedures remain desirable.

### SUMMARY

Specimen retrieval devices in accordance with the present disclosure include a pouch that is selectively detachable from the device. In embodiments, a specimen retrieval device of the present disclosure includes a tubular body defining a longitudinal bore, the tubular body having a proximal portion, a distal portion, and a hand grip supported on the proximal portion of the tubular body; an inner shaft having a proximal portion, a distal portion, an actuation handle supported on the proximal portion of the inner shaft, and a support member extending from a distal portion of the inner shaft; a specimen bag supported on the support member at the distal portion of the inner shaft, the specimen bag including a body defining an opening that is opened and closed by an interlocking assembly, the interlocking assembly including a first interlocking element on one side of the opening, a second interior interlocking element on the opposite side of the opening, and a slider that is movable along the opening to close the opening; and at least one pull string having a proximal portion and a distal portion affixed to the slider, the pull string being movable to move the slider along the opening to close the opening of the specimen bag.

In some embodiments, the support member includes a pair of resilient fingers which support the specimen bag and open the open end of the specimen bag in a deployed state. The resilient fingers may be positioned adjacent the open end of the specimen bag to open the specimen bag when the specimen retrieval device is in the deployed state.

In other embodiments, the specimen bag is furled about the inner shaft in a non-deployed state.

In embodiments, the slider interlocks the first interlocking element and the second interior interlocking element when moved in a closing direction. In some embodiments, the interlocking assembly includes a tongue and groove closure. In other embodiments, the interlocking assembly is a zipper assembly.

In embodiments, the proximal portion of the pull string extends proximally from the actuation handle. In some embodiments, the at least one pull string includes two pull strings, each pull string having a proximal portion and a distal portion affixed to the slider, each pull string moving through a channel of the inner shaft to move the slider to close the opening of the specimen bag. In other embodiments, the proximal portion of each of the two pull strings extends proximally from the actuation handle.

Methods for utilizing the specimen retrieval devices of the present disclosure are also provided. In embodiments, methods of the present disclosure include introducing into a body opening a specimen retrieval device, the specimen retrieval device including a tubular body defining a longitudinal bore and including a proximal portion and a distal portion. The specimen retrieval device also includes an inner shaft having a proximal portion, a distal portion, an actuation handle supported on the proximal portion of the inner shaft, and a support member extending from a distal portion of the inner shaft, the inner shaft capable of passage through the longitudinal bore of the outer shaft; a specimen bag supported on the support member at the distal portion of the inner shaft, the specimen bag including a body defining an opening that is opened and closed by an interlocking assembly, the interlocking assembly including a first interlocking element on one side of the opening, a second interior interlocking element on the opposite side of the opening, and a slider; and at least one pull string having a proximal portion and a distal portion affixed to the slider, the pull string moving through at least one channel of the inner shaft to move the slider to close the opening of the specimen bag. The method further includes inserting the specimen retrieval device through an incision so the distal portion of the tubular body is in a body cavity; passing the distal portion of the inner shaft through the tubular body so that the distal portion of the inner shaft and the specimen bag are distal to the distal portion of the tubular body; passing a tissue specimen through the opening of the specimen bag into the specimen bag; moving the slider to close the interlocking assembly, thereby closing the opening in the specimen bag; and removing the specimen retrieval device from the body cavity.

In embodiments, the method of the present disclosure further includes breaking up the tissue specimen in the specimen bag prior to removing the specimen retrieval device from the body cavity.

In some embodiments, moving the slider interlocks the first interlocking element and the second interior interlocking element, thereby closing the opening in the specimen bag.

In other embodiments, proximally pulling the pull string moves the slider to close the interlocking assembly.

### BRIEF DESCRIPTION OF THE DRAWINGS

Embodiments of the presently disclosed specimen retrieval device are described hereinbelow with reference to the drawings wherein:
FIG. 1 is a side perspective view of a specimen retrieval device in accordance with an exemplary embodiment of the present disclosure;
FIG. 2 is an enlarged view of the indicated area of detail shown in FIG. 1;
FIG. 3 is a side perspective view showing the assembly of the specimen retrieval device shown in FIG. 1;
FIG. 4 is an exploded view of the specimen retrieval device shown in FIG. 1;
FIG. 5 is a cross-sectional view taken along section line 5-5 of FIG. 1;
FIG. 6 is a side, perspective view of the specimen retrieval device shown in FIG. 1 in use; and
FIG. 7 is an enlarged view of the indicated area of detail shown in FIG. 6.

### DETAILED DESCRIPTION OF THE EMBODIMENTS

The present disclosure provides a specimen retrieval device for use in minimally invasive surgical procedures. As used herein with reference to the present disclosure, minimally invasive surgical procedures encompass laparoscopic procedures, arthroscopic procedures, and endoscopic procedures, and refer to procedures utilizing scopes or similar devices having relatively narrow operating portions capable of insertion through a small incision in the skin.

The aspects of the present disclosure may be modified for use with various methods for retrieving tissue specimens during minimally invasive surgical procedures, sometimes referred to herein as minimally invasive procedures. Examples of minimally invasive procedures include, for example, cholecystectomies, appendectomies, nephrectomies, colectomies, splenectomies, and the like.

The presently disclosed specimen retrieval device will now be described in detail with reference to the drawings in which like reference numerals designate identical or corresponding elements in each of the several views. In this description, the term "proximal" is used generally to refer to that portion of the device that is closer to a clinician, while the term "distal" is used generally to refer to that portion of the device that is farther from the clinician. The term "clinician" is used generally to refer to medical personnel including doctors, surgeons, nurses, and support personnel.

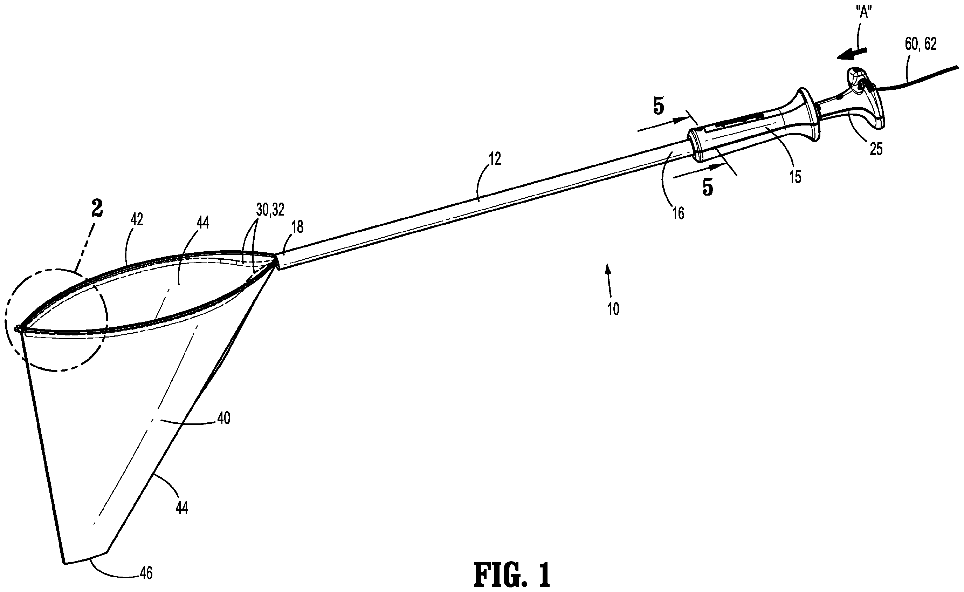

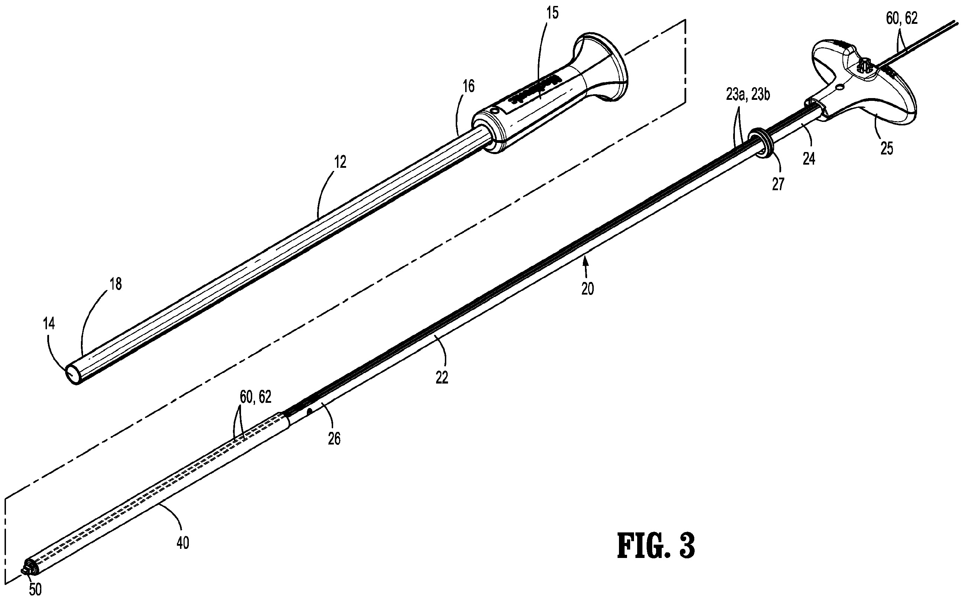

Referring to FIGS. 1-7, and initially with reference to FIGS. 1-4, the specimen retrieval device 10 of the present disclosure includes a tubular body 12 having a proximal portion 16 and a distal portion 18, and defines a longitudinal bore 14 that extends between the proximal portion 16 and the distal portion 18. The tubular body 12 has a hand grip 15 thereon. The specimen retrieval device 10 also includes an inner shaft assembly 20 (FIG. 3) including an inner shaft 22 slidably positioned within the longitudinal bore 14 of the tubular body 12, an actuation handle 25 secured to a proximal portion 24 of the inner shaft 22, and a specimen bag 40 (FIG. 1) supported on a distal portion 26 of the inner shaft 22.

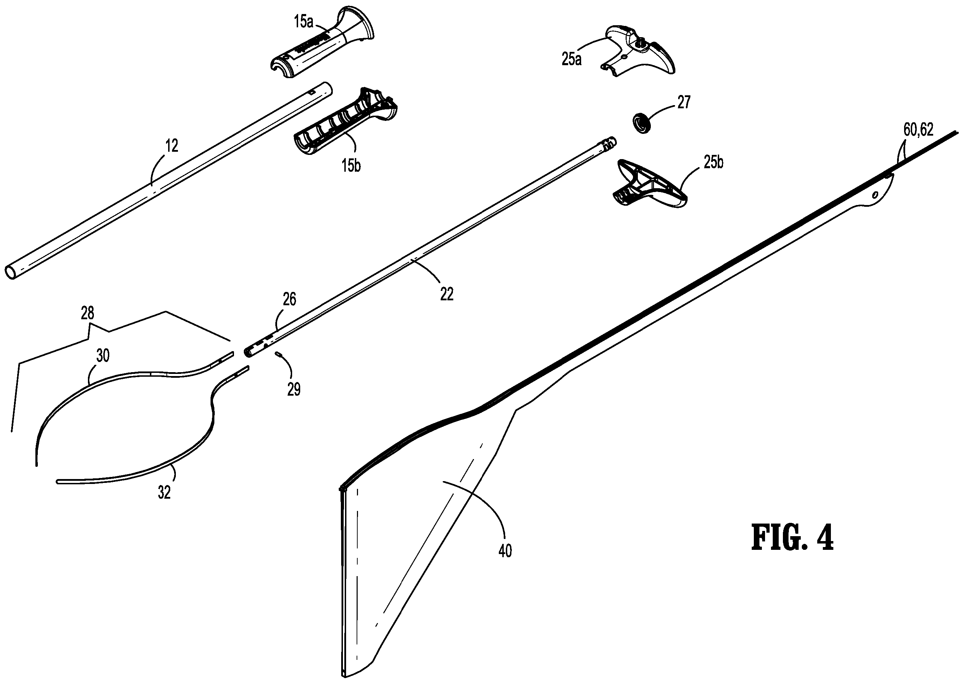

The hand grip 15 on the tubular body 12 may be formed as a unitary component or, as depicted in FIG. 4, as two separate half components 15a, 15b, that are coupled to one another about the tubular body 12 by one or more suitable coupling methods (e.g., one or more suitable adhesives). In the latter instance, an indent/detent configuration (not shown) may be utilized to facilitate coupling the two separate half components on 15a, 15b to one another.

The actuation handle 25 on the inner shaft 22 may likewise be formed as a unitary component or, in embodiments, as depicted in FIG. 4, as two separate half components 25a, 25b that are coupled to one another by one or more suitable coupling methods (e.g., one or more suitable adhesives). An O-ring 27 (FIG. 4) may be secured to the proximal portion 24 of the inner shaft 22. The O-ring 27 is configured to provide adequate clearance between the tubular body 12 and the inner shaft 22 so as to allow unhindered translation of the inner shaft 22 with respect to the tubular body 12 when the inner shaft 22 is translated in the proximal and/or distal directions. Moreover, the O-ring 27 also provides a fluid tight seal to maintain any insufflation gases in a working space, e.g., within a body cavity of a patient.

In an assembled configuration, the hand grip 15 and the actuation handle 25 can be manipulated to facilitate manipulation of the specimen retrieval device 10 and the sliding of the inner shaft 22 within the tubular body 12. More specifically, the hand grip 15 can be grasped by the clinician with one hand and the actuation handle 25 can be grasped by the clinician with the other hand to move the inner shaft 22 within the tubular body 12.

The tubular body 12 and/or the inner shaft assembly 22 of the present disclosure are made of biocompatible materials within the purview of those skilled in the art, in embodiments, polymeric materials. For example, the tubular body 12 and/or the inner shaft assembly 22 may be made of thermoplastic polyurethanes sold under the name PELLETHANE®, which offer flexibility and a wide range of hardness. The tubular body 12 and/or the inner shaft assembly 22, for example, may be fabricated from PELLETHANE® 2363-80A, PELLETHANE® 2363-90A, PELLETHANE® 2363-55D, any combination thereof, or any alternatives within the purview of those skilled in the art.

In some embodiments, the tubular body 12 and the inner shaft assembly 22 are formed of the same material. In other embodiments, the tubular body 12 and the inner shaft assembly 22 are formed of different materials.

The specimen bag 40 includes a body 44 having a generally tubular or elongated configuration that is defined by an openable and closable portion (or mouth) 42 and a closed portion 46. The closable portion 42 defines an opening 45. Alternatively, other specimen bag configurations are envisioned. Referring to FIG. 4, the distal portion 26 of the inner shaft 22 is coupled to a support member 28 that is configured to support the specimen bag 40. In embodiments, the support member 28 includes a pair of resilient fingers 30, 32 that extend distally from the distal portion 26 of the inner shaft 22. In embodiments, the resilient fingers 30, 32 can be integrally formed with the distal portion 26 of the inner shaft 22 such as by molding.

Alternatively, as depicted in FIG. 4, a retention pin 29 may be used to attach the resilient fingers 30, 32 to the distal portion 26 of the inner shaft 22. The resilient fingers 30, 32 are movable from a spaced non-deformed state (FIG. 1) to a deformed state (FIG. 3) to facilitate placement of the specimen bag 40 into the tubular body 12. The resilient fingers 30, 32 return to the non-deformed state when the specimen bag 40 is deployed from the tubular body 12 to open the opening 42 of the specimen bag 40, as described below. Alternately, the specimen bag 40 can be supported on the distal portion 26 of the inner shaft 22 using other fastening or securing techniques.

The body 44 of the specimen bag 40 may be made from any suitable biocompatible material (e.g., nylon, urethane, ripstop nylon or latex) capable of forming a flexible collapsible member, or membrane. In embodiments, the material from which the specimen bag is made is resilient, antistatic, pyrogen-free, non-toxic, and sterilizable. In embodiments, materials used to form the tubular body 12 and/or the inner shaft 22 described above may be used to form the specimen bag 40. In other embodiments, the specimen bag 40 is formed of materials that are different from those used to form the tubular body 12 and/or the inner shaft 22. The specimen bag 40 may be opaque or clear.

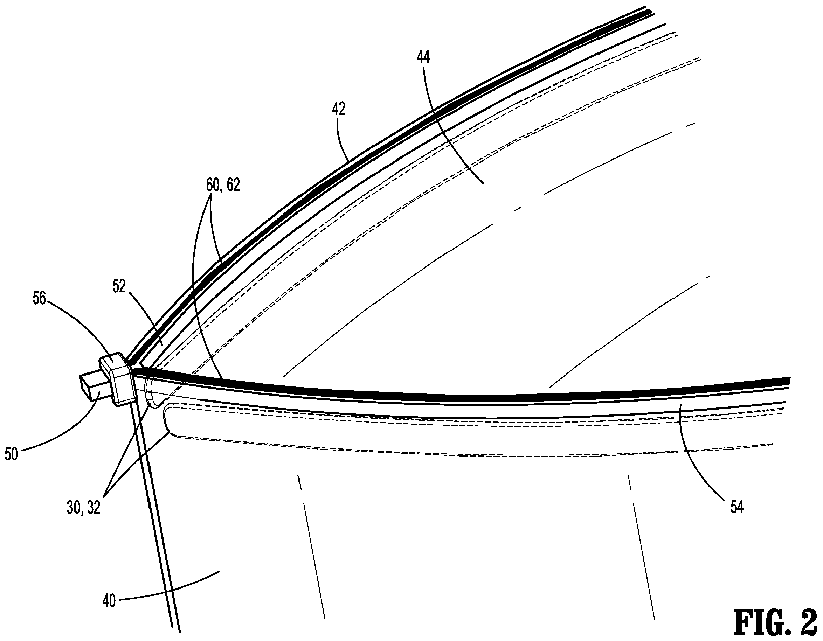

As depicted in FIGS. 1, 2, 6, and 7, the mouth 42 of the specimen bag 40 includes an interlocking assembly 50 which, when opened, forms the opening 45 of the specimen bag 40. The interlocking assembly 50 includes a first interlocking element 52 on one side of the opening 44, and a second interlocking element 54 on the opposite side of the opening 45 (FIG. 2). The first and second interlocking elements 52, 54 include male and female interlocking elements, which can be reversed, or other interlocking configurations can be used.

Returning to FIG. 2, the interlocking assembly 50 also includes a slider 56 mounted on the proximal ends of first and second interlocking elements 52, 54. The slider 56 operates in a conventional manner of separating the first and the second interior interlocking elements 52, 54 when moved in an opening direction and interlocking the first and the second interlocking elements 52, 54 when moved in a closing direction. In some embodiments the interlocking assembly 50 includes a tongue and groove closure, similar to that found on ZIPLOC™ storage bags. In other embodiments, the interlocking assembly may be in the form of a zipper configuration, sometimes referred to herein, in embodiments, as a zipper assembly.

In embodiments, the mouth 42 of the specimen bag 40 has pull strings 60, 62 attached thereto, as well as resilient fingers 30, 32 attached thereto. In other embodiments, the resilient fingers 30, 32 and the pull strings 60, 62 may be received in a cuff (not shown) formed at the mouth 42 of the specimen bag 40. The cuff may be formed on the specimen bag 40 by any suitable method. In embodiments, for example, a top portion of the specimen bag 40 may be folded into an interior thereof or onto an exterior thereof and, subsequently, glued thereto to form the cuff.

Referring to FIGS. 2-5, the inner shaft 22 includes two channels 23a, 23b (FIG. 3) allowing for passage of the pull strings 60, 62 (FIGS. 2 and 3), or similar devices through or along the inner shaft 22. The pull strings 60, 62 are attached to the slider 56 (FIG. 2) of the interlocking assembly 50. The pull strings 60, 62 pass from the slider 56 through the channels 23a, 23b on the inner shaft 22 and pass through the actuation handle 25 that remains outside the body. In alternate embodiments, a single pull string (not shown) may pass through a single channel (not shown) of the inner shaft 22 and be attached to the slider 56. Where the specimen retrieval device 10 includes the pull strings 60, 62 to close the interlocking assembly 50, the slider 56 is positioned at a distal location upon deployment of the specimen bag 40 into a patient.

As depicted in FIG. 3, the specimen bag 40 may be furled about a distal portion of the inner shaft 22 for insertion into the longitudinal bore 14 of the tubular body 12. More specifically, the inner shaft 22 may be rotated either clockwise or counter-clockwise prior to the inner shaft 22 being introduced into the tubular body 12, so that the specimen bag 40 is furled about the support member 28 at the distal portion 26 of the inner shaft 22. Thereafter, the specimen bag 40 can be introduced into the longitudinal bore 14 of the tubular body 12. The specimen retrieval device 10 is then ready to be introduced into a patient. FIG. 3 shows the furled specimen bag 40 prior to insertion into the tubular body 12.

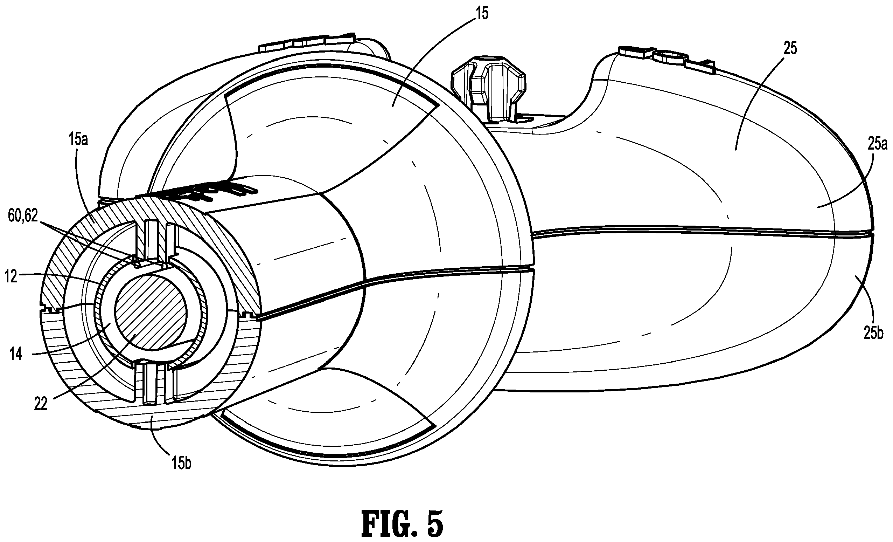

FIG. 5 is a cross-sectional view of the specimen retrieval device 10 of the present disclosure, showing the inner shaft 22 within the longitudinal bore 14 of the tubular body 12 at the distal portion of the hand grip 15. Both the hand grip 15 of the tubular body 12 and the actuation handle 25 of the inner shaft 22 are visible. FIG. 5 shows the inner shaft 22 positioned within the longitudinal bore 14 of the tubular body 12, as well as pull strings 60, 62 within hand grip 15.

In use, the tubular body 12 of the specimen retrieval device 10 can be inserted through an incision (not shown) with the specimen bag 40 furled about the inner shaft 22 and positioned within the tubular body 12 to position the specimen bag 40 in a body cavity adjacent a surgical site. When the tubular body 12 is properly positioned, the clinician can grip the hand grip 15.

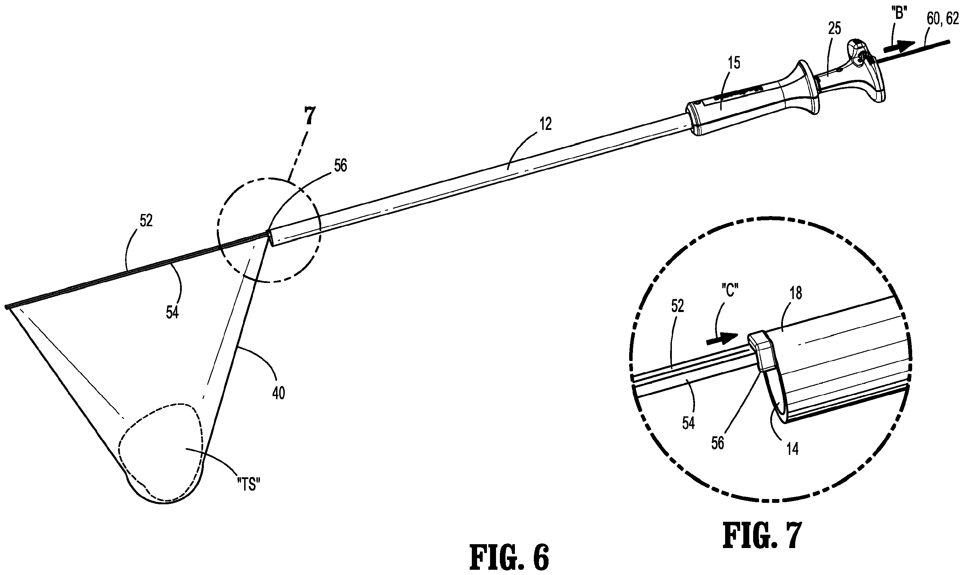

Turning to FIGs. 1 and 6, the clinician then pushes the actuation handle 25 on the proximal portion 24 of the inner shaft 22 distally in relation to the hand grip 15 and the tubular body 12, in the direction indicated by arrow "A" in FIG. 1, so the distal portion 26 of the inner shaft 22, including the specimen bag 40, exits the distal portion 18 of the tubular body 12. Once the specimen bag 40 has exited the tubular body 12, the resilient fingers 30, 32 (FIG. 4) return to their non-deformed state, thereby opening the mouth 42 of the specimen bag 40 to ensure the specimen bag 40 is deployed.

As shown in FIGs. 1 and 6, when the specimen bag 40 is unfurled, the resilient fingers 30, 32, return to their non-deformed state to open the mouth 42 of the specimen bag 40 so that the specimen bag 40 is in a fully deployed position capable of receiving a tissue specimen "TS" (FIG. 6) therein.

As depicted in FIGs. 6 and 7, after a tissue specimen "TS" has been placed in the specimen bag 40, the pull strings 60, 62 are pulled proximally (indicated by arrow "B" in FIG. 6) to pull the pull strings 60, 62 proximally, thus pulling the slider 56 proximally (indicated by arrow "C" in FIG. 7) and thereby joining the interlocking elements 52, 54 of the interlocking assembly 50. The joining of the interlocking elements 52, 54 of the interlocking assembly 50 closes the mouth 42 of the specimen bag 40. In some embodiments, a pull ring (not shown) may be affixed to the proximal portion of the pull strings 60, 62 and used to assist in pulling the pull strings 60, 62.

Kits of the present disclosure may include both the specimen retrieval device described above, as well as trocars, graspers, vacuum sources (tubes), combinations thereof, and the like. In some embodiments, these additional devices, such as graspers and/or vacuum sources, may be used to break up the tissue specimen in the specimen bag prior to removing the specimen retrieval device from the body cavity.

Once the specimen retrieval device of the present disclosure has been removed from the patient's body, any tissue specimen "TS" may be removed from the specimen bag 40 for further examination and the specimen bag 40 may be discarded.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto. Additionally, it is envisioned that the elements and features illustrated or described in connection with one exemplary embodiment may be combined with the elements and features of another without departing from the scope of the present disclosure, and that such modifications and variations are also intended to be included within the scope of the present disclosure. Accordingly, the invention is not to be limited by what has been particularly shown and described, except as indicated by the appended claims.

The invention may be described by reference to the following numbered paragraphs:-
1. A specimen retrieval device, comprising:
   a tubular body defining a longitudinal bore, the tubular body having a proximal portion, a distal portion, and a hand grip supported on the proximal portion of the tubular body;
   an inner shaft having a proximal portion, a distal portion, an actuation handle supported on the proximal portion of the inner shaft, and a support member extending from a distal portion of the inner shaft;
   a specimen bag supported on the support member at the distal portion of the inner shaft, the specimen bag including a body defining an opening that is opened and closed by an interlocking assembly, the interlocking assembly including a first interlocking element on one side of the opening, a second interior interlocking element on the opposite side of the opening, and a slider that is movable along the opening to close the opening; and
   at least one pull string having a proximal portion and a distal portion affixed to the slider, the pull string being movable to move the slider along the opening to close the opening of the specimen bag.
2. The specimen retrieval device of paragraph 1, wherein the support member includes a pair of resilient fingers which support the specimen bag and open the open end of the specimen bag in a deployed state.
3. The specimen retrieval device of paragraph 2, wherein the resilient fingers are positioned adjacent the open end of the specimen bag to open the specimen bag when the specimen retrieval device is in the deployed state.
4. The specimen retrieval device of paragraph 1, wherein the specimen bag is furled about the inner shaft in a non-deployed state.
5. The specimen retrieval device of paragraph 1, wherein the slider interlocks the first interlocking element and the second interior interlocking element when moved in a closing direction.
6. The specimen retrieval device of paragraph 1, wherein the interlocking assembly includes a tongue and groove closure.
7. The specimen retrieval device of paragraph 1, wherein the interlocking assembly is a zipper assembly.
8. The specimen retrieval device of paragraph 1, wherein the proximal portion of the pull string extends proximally from the actuation handle.
9. The specimen retrieval device of paragraph 1, wherein the at least one pull string includes two pull strings, each pull string having a proximal portion and a distal portion affixed to the slider, each pull string moving through a channel of the inner shaft to move the slider to close the opening of the specimen bag.
10. The specimen retrieval device of paragraph 8, wherein the proximal portion of each of the two pull strings extends proximally from the actuation handle.
11. A method comprising:
   introducing into a body opening a specimen retrieval device including:
      a tubular body defining a longitudinal bore and including a proximal portion and a distal portion;
      an inner shaft having a proximal portion, a distal portion, an actuation handle supported on the proximal portion of the inner shaft, and a support member extending from a distal portion of the inner shaft, the inner shaft capable of passage through the longitudinal bore of the outer shaft;
      a specimen bag supported on the support member at the distal portion of the inner shaft, the specimen bag including a body defining an opening that is opened and closed by an interlocking assembly, the interlocking assembly including a first interlocking element on one side of the opening, a second interior interlocking element on the opposite side of the opening, and a slider; and
      at least one pull string having a proximal portion and a distal portion affixed to the slider, the pull string moving through at least one channel of the inner shaft to move the slider to close the opening of the specimen bag;
   inserting the specimen retrieval device through an incision so the distal portion of the tubular body is in a body cavity;
   passing the distal portion of the inner shaft through the tubular body so that the distal portion of the inner shaft and the specimen bag are distal to the distal portion of the tubular body;
   passing a tissue specimen through the opening of the specimen bag into the specimen bag;
   moving the slider to close the interlocking assembly, thereby closing the opening in the specimen bag; and
   removing the specimen retrieval device from the body cavity.
12. The method of paragraph 11, further including breaking up the tissue specimen in the specimen bag prior to removing the specimen retrieval device from the body cavity.
13. The method of paragraph 11, wherein the support member includes a pair of resilient fingers which support the specimen bag and open the open end of the specimen bag in a deployed state.
14. The method of paragraph 11, wherein the slider interlocks the first interlocking element and the second interior interlocking element, thereby closing the opening in the specimen bag.
15. The method of paragraph 11, wherein proximally pulling the pull string moves the slider to close the interlocking assembly.

## Claims

1. A specimen retrieval device, comprising:
a tubular body defining a longitudinal bore, the tubular body having a proximal portion, a distal portion, and a hand grip supported on the proximal portion of the tubular body;
an inner shaft having a proximal portion, a distal portion, an actuation handle supported on the proximal portion of the inner shaft, and a support member extending from a distal portion of the inner shaft;
a specimen bag supported on the support member at the distal portion of the inner shaft, the specimen bag including a body defining an opening that is opened and closed by an interlocking assembly, the interlocking assembly including a first interlocking element on one side of the opening, a second interior interlocking element on the opposite side of the opening, and a slider that is movable along the opening to close the opening; and
at least one pull string having a proximal portion and a distal portion affixed to the slider, the pull string being movable to move the slider along the opening to close the opening of the specimen bag.

2. The specimen retrieval device of claim 1, wherein the support member includes a pair of resilient fingers which support the specimen bag and open the open end of the specimen bag in a deployed state.

3. The specimen retrieval device of claim 2, wherein the resilient fingers are positioned adjacent the open end of the specimen bag to open the specimen bag when the specimen retrieval device is in the deployed state.

4. The specimen retrieval device of any preceding claim, wherein the specimen bag is furled about the inner shaft in a non-deployed state.

5. The specimen retrieval device of any preceding claim, wherein the slider interlocks the first interlocking element and the second interior interlocking element when moved in a closing direction.

6. The specimen retrieval device of any preceding claim, wherein the interlocking assembly includes a tongue and groove closure.

7. The specimen retrieval device of any preceding claim, wherein the interlocking assembly is a zipper assembly.

8. The specimen retrieval device of any preceding claim, wherein the proximal portion of the pull string extends proximally from the actuation handle.

9. The specimen retrieval device of any preceding claim, wherein the at least one pull string includes two pull strings, each pull string having a proximal portion and a distal portion affixed to the slider, each pull string moving through a channel of the inner shaft to move the slider to close the opening of the specimen bag.

10. The specimen retrieval device of claim 8, wherein the proximal portion of each of the two pull strings extends proximally from the actuation handle.
